# EUROPEAN PATENT SPECIFICATION

(11) **EP 1 276 433 B1**
(45) Date of publication and mention of the grant of the patent: **14.08.2013**
(21) Application number: 01925004.2
(22) Date of filing: 13.04.2001
(51) Int. Cl.: A61C 3/00, A61C 7/00

(54) **METHOD FOR MAKING A TEMPLATE FOR PLACEMENT OF ORTHODONTIC APPARATUS**
VERFAHREN ZUR HERSTELLUNG EINER SCHABLONE ZUR EINSETZUNG EINES ORTHODONTISCHEN GERÄTS
PROCEDE DE FABRICATION D'UN GABARIT PERMETTANT DE POSER UN APPAREIL ORTHODONTIQUE

(30) Priority: 19.04.2000 US 552189; 19.04.2000 US 552190; 19.04.2000 US 552191; 28.04.2000 US 560127; 28.04.2000 US 560129; 28.04.2000 US 560130
(43) Date of publication of application: 22.01.2003
(73) Proprietor: OraMetrix, Inc., Richardson, TX 75082 (US)
(72) Inventor: RUBBERT, Rüdger, 12101 Berlin (DE); WEISE, Thomas, 10965 Berlin (DE); RIEMEIER, Friedrich, 10557 Berlin (DE); SACHDEVA, Rohit, Plano, TX 75093 (US); PLACKE, Michael, Plano, TX 75023 (US); JOHNSON, Matthew, Dallas, TX 75206 (US)
(74) Representative: Stevens, Jason Paul
(86) International application number: PCT/US2001/012105
(87) International publication number: WO 2001/085047

(56) References cited:
- US-A- 4 160 322
- US-A- 4 551 096
- US-A- 5 683 243
- US-A- 5 863 198
- US-A- 5 879 158

## Description

### Field of the Invention

This invention relates generally to the practice of orthodontics and in particular to a method for generating an orthodontic template for placing orthodontic apparatus.

### Background of the Invention

Orthodontics is known to be the practice of manipulating a patient's teeth to provide better function and appearance. In general, brackets are bonded to a patient's teeth and coupled together with an arch wire. The combination of the brackets and wire provide a force on the teeth causing them to move. Once the teeth move to a desired location and are held in place for a certain period of time, the body adapts bone and tissue to maintain the teeth in the desired location. To further assist in retaining the teeth in the desired location, the patient may be fitted with a retainer.

To achieve tooth movement, orthodontists utilize their expertise to first determine a three-dimensional mental image of the patient's physical orthodontic structure and a three-dimensional mental image of a desired physical orthodontic structure for the patient, which may be assisted through the use of x-rays and/or models. Based on these mental images, the orthodontist further relies on his or her expertise to place the brackets and/or bands on the teeth and then manually bends (i.e., shape) the arch wire such that a force is exerted on the teeth to reposition the teeth into the desired physical orthodontic structure. As the teeth move towards the desired location, the orthodontist makes continual judgments as to the progress in the treatment, the next step in the treatment (e.g., new bends in the arch wire, repositioning or replacing brackets, is headgear required, etc.) and the success of the previous step.

In general, the orthodontist makes manual adjustments to the arch wire and/or replaces or repositions brackets based on his or her own expert opinion. Unfortunately, in the oral environment, it is impossible for a human being to accurately develop a three-dimensional mental image of an orthodontic structure due to the limitations of human sight and the physical structure of a human mouth. In addition, it is humanly impossible to accurately estimate three-dimensional wire bends (with an accuracy within a few degrees) and to manually apply such bends to a wire. Further it is humanly impossible to determine an ideal bracket location to achieve the desired orthodontic structure based on mental images. It is also extremely difficult to manually place brackets in the estimated ideal location, to control bonding agent thickness, ligation forces, manufacturing tolerances, and biological changes. Accordingly, orthodontic treatment is an iterative process requiring multiple wire changes, with the type, success, and speed of treatment being very much dependent on the orthodontist's motor skills and diagnostic expertise. As a result of multiple wire changes, patient discomfort is increased as well as treatment costs. As one would expect, the quality of care varies greatly from orthodontist to orthodontist, as does the time to treat a patient.

As described, the practice of orthodontics is very much an art, relying on the expert opinion and judgment of the orthodontist. In an effort to shift the practice of orthodontics from an art to a science, many innovations have been developed. For example, U.S. Patent 5,518,397 issued to Andreiko, et. al, provides a method of forming an orthodontic brace. Such a method includes obtaining a model of the teeth of a patient's mouth and a prescription of desired positioning of such teeth. The contour of the teeth of the patient's mouth is determined from the model. Calculations of the contour and the desired positioning of the patient's teeth are then made to determine the geometry (e g., grooves or slots) to be provided. Custom brackets including a special geometry have been created for receiving an arch wire to form an orthodontic brace system. Such geometry is intended to provide for the disposition of the arched wire on the bracket in a progressive curvature of a horizontal plane and a substantially linear configuration in a vertical plane. The geometry of the bracket is altered (e.g., by cutting grooves into the bracket at individual positions and angles and with particular depth), in accordance with such calculations of the geometry of the patient's teeth. In such a system, the brackets are customized to provide three-dimensional movement of the teeth once the wire, which has a two-dimensional shape (i.e., linear shape in the vertical plane and curvature in the horizontal plane) is applied to the brackets.

Unfortunately, the current innovations to change the practice of orthodontics from an art to a science have only made limited progress. This is due to, but not restricted to, the brackets being the focal point for orthodontic manipulation. By having the brackets as the focal point, placement of each bracket on a corresponding tooth is critical. Since each bracket includes a custom sized and positioned wire retaining groove, a misplacement of a bracket by a small amount (e.g., an error vector having a magnitude of a millimeter or less and an angle of a few degrees or less) can cause a different force system (i.e., magnitude of movement and direction of movement) than the desired force system to be applied to the teeth. As such, the tooth will not be repositioned to the desired location.

In general, there are two methods for applying brackets to teeth: an indirect method and a direct method. For the indirect method, a tooth impression model is created in the patient's mouth using a hardening material. The tooth impression model is then used to create a model of the teeth. Brackets are then manually placed on, and temporarily bonded to, the model of the teeth. A transfer tray is then fabricated by taking an impression of the model of the teeth with the brackets installed. Once the transfer tray is fabricated, brackets are placed, therein and a bonding agent is applied to the bonding pad of each bracket.

Once an orthodontist has a transfer tray with brackets installed, the orthodontist manually positions the tray into the patient's mouth to place the brackets on the patient's teeth. Once the orthodontist believes the brackets are positioned properly, (s)he bonds the brackets to the teeth. Unfortunately, this manual process has limited accuracy due to human limitations. As such, it is extremely difficult, if not impossible, for the orthodontist to position all of the brackets in an ideal location with known bonding agent thickness, slot position, etc. or verify such placements.

The direct bonding method is, as the name implies, a method where the brackets are directly bonded to the patient's teeth without the use of a transfer tray. The direct bonding method is typically less accurate than the indirect bonding method since the entire process is done manually without any mechanical assistance. Furthermore, it is difficult to manually judge the location of brackets during placement.

U.S. Patent No. 5.366.478 and U.S. Patent No USS, 683, 243 both issued to Andreiko, et. al provides an indirect bonding method that forms jigs for custom placement of orthodontic appliances on teeth. In general, the '478 patent teaches that each orthodontic template is provided with a surface conforming to the contour of the tooth to which they are to be mounted. Another surface of the orthodontic template, or jig, engages the bracket to hold it in the proper position and orientation for mounting to the tooth and spaced in relation to the contour surface to precisely locate the orthodontic template on the tooth. The orthodontic templates are particularly useful in positioning brackets of custom appliances desired to the individual anatomy of the patient and requiring custom positions of the brackets on the teeth. While the '478 patent discloses a method for forming a jig, auch jig utilisation still keeps the bracket as the local point of the orthodontic treatment and provides no feedback mechanism regarding actual placement of the bracket. Further, the '478 patent does not allow for variables associated with tooth movement such as static, dynamic, or psychosocial mechanical and/or biological changes. U.S. Patents 6.011,405 and 5,542,842 teach indirect bonding appraches, but suffer the same limitations as the '478 patent.

To achieve optimal orthodontic treatment, the force system applied to each tooth must be optimal. The scientific approach as prevlously discussed, however, relies on initial diagnostic and orthodontic apparatus design without any means for verification once the initial diagnostics is complete. Once the orthodontic apparatus is designed, the accuracy of all subsequent patient treatment relies on accurate placement of the brackets of the orthodontic structure. If the bracket is not of an optimal design or if the bracket is misplaced, the desired force system on the tooth will not be obtained. As such, orthodontics treatment will not be as accurate as desired and thus will be less than optimal.

Therefore, a need exists for a method for generating an orthodontic template that assists in the placement of an orthodontic apparatus without the limitations of current jig and transfer tray designs. Further, a need exists for a method for providing feedback when placing brackets on teeth to ensure proper placement. In addition, to further enhance the accuracy of orthodontics treatment a need exists for a method that enables optimized arch wire receptacle designs.

### Brief Description of the Drawings

Figure 1 illustrates a schematic block diagram of an orthodontic service system in accordance with the present invention;
Figure 2 illustrates a graphical representation of a three-dimensional digital model of an actual orthodontic structure in accordance with the present invention;
Figure 3 illustrates a graphical representation of an actual bracket placement versus ideal bracket placement in accordance with the present invention;
Figure 4 illustrates a graphical representation of projected bracket placement in accordance with the present invention;
Figure 5 illustrates a graphical representation of an orthodontic apparatus applied to teeth in accordance with the present invention;
Figure 6 illustrates an isometric view of a bracket having a generic wire retention receptacle in accordance with the present invention;
Figure 7 illustrates an isometric view of a bracket including a specific wire retention receptacle in accordance with the present invention;
Figure 8 illustrates a graphical representation of bracket undercut correction in accordance with the present invention;
Figure 9 illustrates a three-dimensional digital model of a tooth mounting apparatus in accordance with the present invention;
Figure 10 illustrates a graphical representation of a three-dimensional model of a bracket, or tooth mounting apparatus, being applied to a tooth in accordance with the present invention;
Figure 11 illustrates a graphical representation of a orthodontic template in accordance with the present invention;
Figure 12 illustrates a graphical representation of an alternate orthodontic template in accordance with the present invention;
Figure 13 illustrates a graphical representation of another orthodontic template in accordance with the present invention;
Figure 14 illustrates a graphical representation of bracket undercut correction in accordance with the present invention;
Figure 15 illustrates a graphical representation of a tooth mold and an over-mold orthodontic template in accordance with the present invention;
Figure 16 illustrates a logic diagram of a method for generating a orthodontic template in accordance with the present invention;
Figure 17 illustrates a logic diagram of an alternate method for generating a orthodontic template in accordance with the present invention;
Figure 18 illustrates a logic diagram of a method for bonding a bracket to a tooth in accordance with the present invention;
Figure 19 illustrates a logic diagram of an alternate method for bonding a bracket to a tooth in accordance with the present invention;
Figure 20 illustrates a logic diagram of a method for arch wire receptacle optimization in accordance with the present invention; and
Figure 21 illustrates a logic diagram of an alternate method for arch wire receptacle optimization in accordance with the present invention.

### Detailed Description of Preferred Embodiments

Generally, the present invention provides a method for generating an orthodontic template that assists in the placement of an orthodontic apparatus. Such a method includes processing that begins by obtaining a digital model of an orthodontic structure of an orthodontic patient. Such a model may be obtained in accordance with co-pending patent application entitled "Method and Apparatus for Producing a Three-Dimensional Image of an Orthodontic Structure" having a filing date of November 30,1999 and a serial number of 09/451, 637 and a publication number US 6 471 512. The processing continues by obtaining a selection of one of a plurality of orthodontic apparatuses for the orthodontic structure to produce a selected orthodontic apparatus. The plurality of orthodontic apparatuses may be stored in a database and designed in accordance with co-pending patent application entitled "Method and Apparatus for Designing an Orthodontic Apparatus to Provide Tooth Movement" having a filing date of November 30,1999 and having a serial number of 091451 ,564 and a publication number US 6 350 120. The processing then continues by obtaining a digital model of placement of the selected orthodontic apparatus on the digital model of the orthodontic structure. The processing then continues by retrieving a digital image of a tooth mounting apparatus (e. g., a bracket, a band, a head gear tube, etc.) of the selected apparatus for a given tooth. The processing then continues by generating a orthodontic template for holding a physical embodiment of the tooth mounting apparatus based on the digital image of the tooth mounting apparatus, the digital model of the placement, and at least a portion of the digital model of the orthodontic structure. With such a method, an orthodontic template may be designed without the bracket being the focal point of orthodontic manipulation and thus avoiding the limitations of prior art embodiments. As such, the present invention offers, among other advantages, the ability to iteratively and interactively simulate dynamic/static orthodontic process to optimize bracket placement and the corresponding design of the orthodontic template; the ability to have the orthodontist manufacture the orthodontic template or a portion thereof; the ability to select material characteristics for the template; and the ability to allow the orthodontist to select any orthodontic apparatus and to accurately make an orthodontic structure for the selected orthodontic apparatus and the given patient.

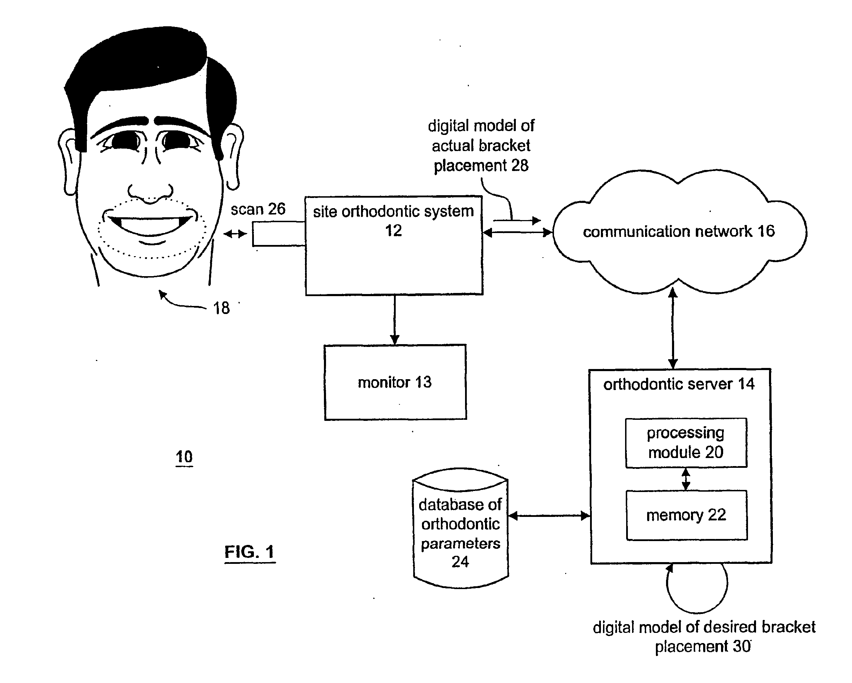

The present invention can be more fully described with Figures 1 through 12. Figure 1 illustrates a schematic block diagram of an orthodontic servicing system 10 that includes a site orthodontic system 12, an orthodontic server 14, a communication network 16, and a database of orthodontio parameters 24. In operation, the site orthontic system 12 scans 26 the patient's 18 arthadontic structure (i.e., teeth, gums, lips, upper and lower arches, and/or other facial features). In one preferred embodiment, the scanning is performed with the brackets temporarily installed. Note that the scanning may be done by ultrasound, laser, light refraction, and/or video imaging in two-dimensional or three-dimensional mode based on the therapeutic needs. The site orthodontic system 12 converts the scanned images of the orthodontics structure of the patient to use a digital model of the actuel orthodontic structure 28. The orthodontic server 14 receives the digital model of the actual orthodontic structure 28 via the communication network 16. The communication network 16 may be a direct connection, the internet, local area network, wide area network, wide area network, and/or any device that enables the transference of digital information from one computing type system to another. Note that a specific embodiment of three-dimensional scanning is described In U S. Application No.09/560,641 filed on April 28, 2000, and published as US 6512994.

The orthodontic server 14 includes a processing module 20 and memory 22. The processing module 20 may be a single processing device or a plurality of processing devices. Such a processing device may be a microprocessor. microcomputer, microcontroller, digital signal processor, central processing unit. state machine, logic circuitry, and/or any device that manipulates signals (eg., analog and/or digital) based on operational instructions. The memory 22 may be a single memory device or a plurality of memory devices. Such a memory device may be a read-only memory, random access memory, floppy disk memory, hard drive memory, system memory, flash memory, and/or any device that stores digital information. Note that when the processing model 20 implements one or more of its functions via a state machine or logic circuitry, the memory storing the corresponding operational instructions is embedded within the circuitry comprising the state machine and/or logic circuitry.

The orthodontic server 14 generates a three-dimensional digital model of the desired orthodontic structure 30 in accordance with the teachings of co-pending U.S. Patent Application No. 09/451,609, published as US 6250918, entitled "Method and Apparatus for Determining Tooth Movement in accordance with an Ideal Orthodontic Structure" filed on November 30,1999 and with co-pending U.S. Patent Application No. 09/451,564, entitled "Method and Apparatus for Designing an Orthodontic Apparatus to Provide Tooth Movement" filed on November 30,1999, from the digital model of the actual orthodontic structure 28 and orthodontic parameters contained in the database of orthodontic parameters 24. To achieve this, the processing module 20, via operational instructions stored in memory 22, performs the processing steps which are discussed in greater detail in co-pending U. S. Patent Application No. 09/452,031 entitled "Method and Apparatus for Generating a Desired Three-Dimensional Digital Model of an Orthodontic Structure" having a filing date of November 30,199 and published as US 6431870. For a more detailed discussion of the site orthodontic system 12, the orthodontic server 14 and the database of orthodontic parameters 24 refer to co-pending U.S. Patent Application No. 09/451,637 entitled "Method and Apparatus for Determining and Monitoring Orthodontic Treatment" having a filing date of November 30,199 and published as US 6471512 co-pending U.S. Patent Application No. 09/451,560 entitled "Method and Apparatus for Treating an Orthodontic Patient" filed on November 30,1999, and published as US 6540512 and co-pending U.S. Patent Application No. 09/452,038 entitled "Method and Apparatus for Site Treatment of an Orthodontic Patient" having a filing date of November 30,199999 and published as US 6315553.

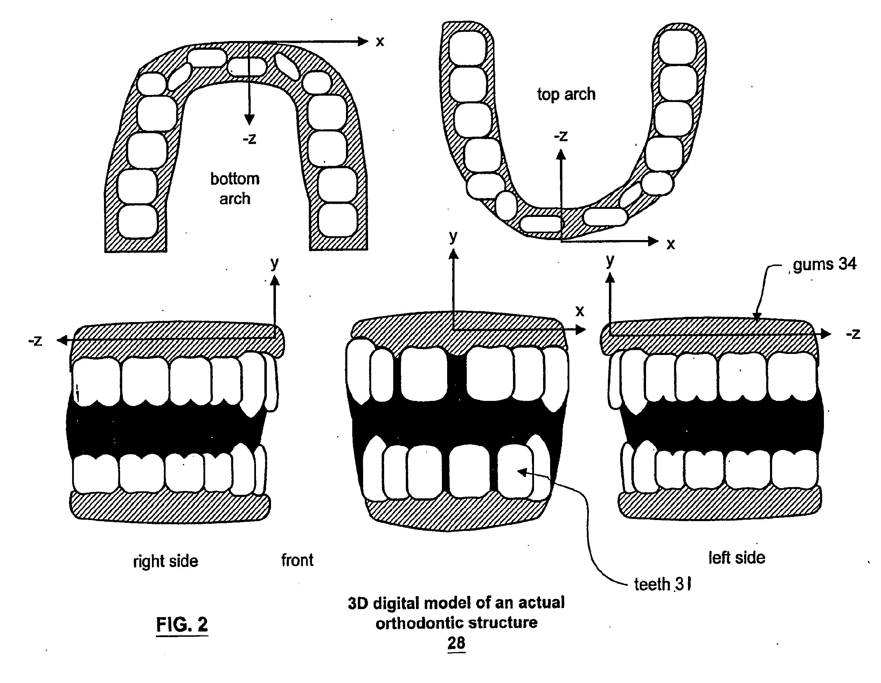

Figure 2 illustrates a graphical representation of the three-dimensional digital model of an actual orthodontic structure 28. As shown, the orthodontic structure is mapped to X, Y, Z coordinate system. For a detailed discussion of the mapping of the digital model to X, Y, Z coordinate system refer to co-pending patent application entitled "Method and Apparatus for Producing a Three-Dimensional Image of an Orthodontic Patient" filed on November 30, 1999 and having a serial number of 09/452,034. As shown, the three-dimensional model of the actual orthodontic structure 28 includes surface images of the teeth 31 and gums 34. The three-dimensional model may further include surface images of the bone structure, lips and other soft facial tissues. The generation of the three-dimensional digital model of the actual orthodontic structure is further described in the aforementioned patent application in this paragraph.

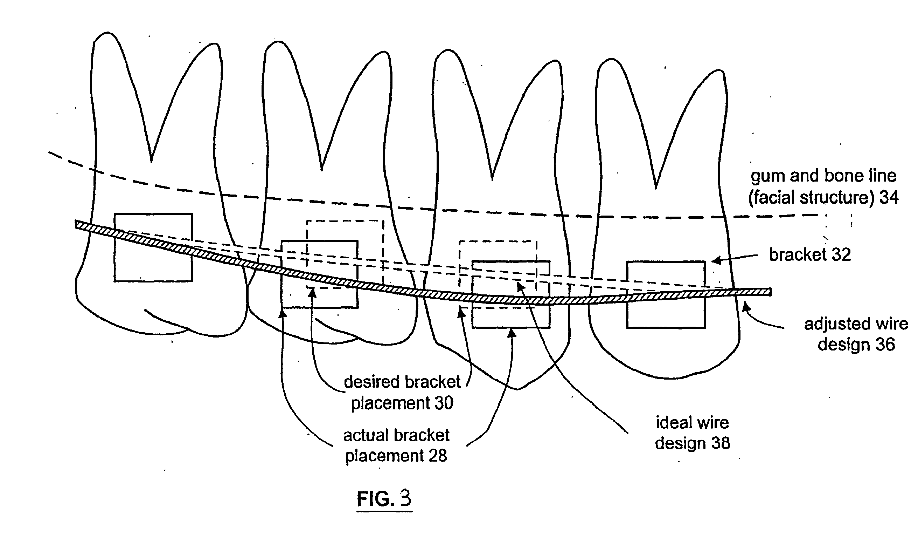

Figure 3 illustrates a graphical representation of the actual placement of brackets and arch wire on a patient's teeth. Note that the wire will not be installed until the brackets have been placed and secured. The illustration also includes a desired bracket placement 30 in comparison to the actual bracket placement 28. As shown, even with the use of a jig or other positioning mechanisms, the actual bracket placement 28 may not correspond to the desired bracket placement 30. By placing the brackets on the tooth and, prior to permanently securing the brackets to the teeth, the actual bracket placement is scanned. The scanned information is then converted to a digital model that may be utilized to compare the actual bracket placement with the desired bracket placement. If an error exists, as shown in Figure 3, the practitioner may re-orientate the bracket placement such that the actual bracket placement coincides with the desired bracket placement. Once the bracket has been placed, the arch wire 36 or 38 is installed to produce the desired force system on the teeth. As shown, the orthodontic apparatus includes the bracket and arch wire and is mounted on the teeth below the gum and bone line (facial structure) 34. Note that the term bracket, as used herein, includes a bracket, a band, head gear tube, or any device used to mount onto a patient's tooth for reception of a displacement apparatus such as an arch wire, rubber band, and/or head gear.

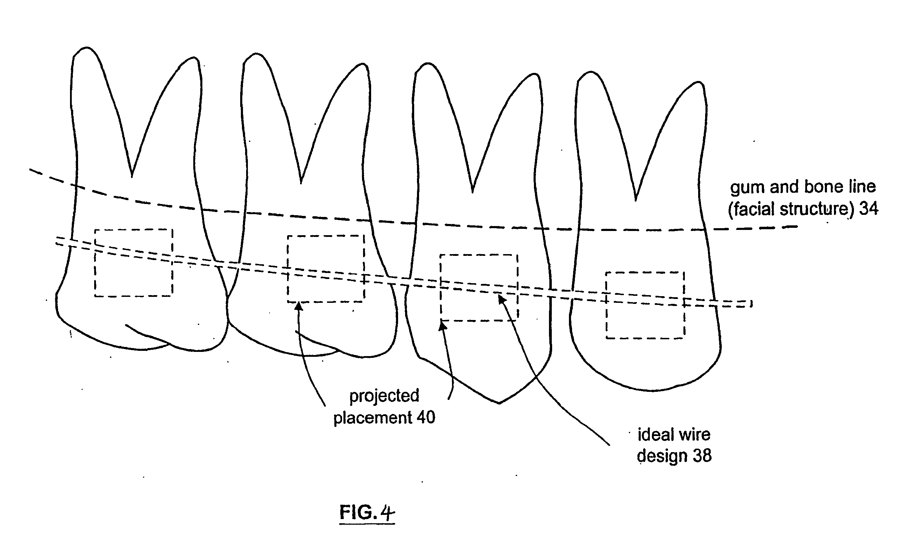

Figure 4 illustrates an alternative mechanism for accurately placing the brackets. As discussed with respect to Figure 3, a placement device, such as a jig, may be utilized to actually place the brackets on the teeth and then scanned to determine whether the actual positioning is in the desired location. Note that the scanning provides a feedback loop. Further note that the feedback of the actual placement may be achieved by digitizing discrete points on the bracket with ultrasound, light scanning, laser, or any other scanning method. If not, the brackets are repositioned until the desired location is achieved. Alternatively, as shown in Figure 4, a projection 40 may be projected on the teeth to illustrate the desired location. The scanning tool in the site orthodontic system 12 may include a projector such that the orthodontic structure, (i.e., teeth, gums, etc.), are displayed on monitor 13 enabling the practitioner to monitor the positioning of the brackets in accordance with the projection 40. Thus, the practitioner is provided a feedback system to ensure the proper placement of the brackets on the teeth. Note that the projection 40 may include a single projection of the outline of the bracket, or a bull's eye type pattern to center the bracket on the tooth. Further note that a combination of the illustration of Figures 3 and 4 may be utilized. For example, the bracket may be positioned in accordance with projection and then subsequently scanned utilizing the illustration of Figure 3 to verify accurate positioning. Still further note that verification may be done by scanning the patient's orthodontic structure directly, scanning a mold of the patient's orthodontic structure, and/or scanning a stereo-lithography of the patient's orthodontic structure, with the orthodontic appliances printed thereon, as a positive or negative image. Figure 4 illustrates a graphical representation of a patient's teeth having an orthodontic apparatus attached thereto via a digital model. The orthodontic apparatus includes a plurality of digitally placed brackets 40 and an ideal arch wire 38. Note that the term bracket, as used herein, includes a bracket, a band, head gear tube, or any device used to mount onto a patient's tooth for reception of a displacement apparatus such as an arch wire, rubber band, and/or head gear. As shown, the brackets 40 and arch wire 38 are positioned below the gum and bone line 34. Each tooth, via the digitally placed brackets 40 and the ideal arch wire 38 will experience a force system causing the tooth to be repositioned in an optimal treatment manner. Optimal treatment includes factors such as shortest displacement distance, minimal number of wire changes, selection of brackets, cost, treatment time, patient discomfort tolerance, available appliances, etc. and can be digitally modelled to ensure that the force system is optimal from the outset of the treatment.

The force system that causes a tooth to move from its malocclusion position to its desired position has many contributing biological factors and mechanical factors. For example, the mechanical factors include peeling forces of the bonding agent, shear bond strength, ligation forces, wire characteristics, the type of adhesive used, anatomy of the tooth, bracket geometry, bracket slot geometry, bracket base configuration, arch wire shape, arch wire elasticity, arch wire diameter, and manufacturing tolerances. The biological factors include tooth interference, tooth anatomy, growth of the jaw, eruption of teeth, jaw function, root length, and periodontal characteristics. In accordance with the teachings of the present invention, each of these factors can be digitally simulated to determine a resulting force system. In addition, each contributing factor may be digitally altered to optimize the force system prior to any orthodontic appliances being installed on the patient's orthodontic structure. Note that each contributing factor to the force system may be stored in the database of orthodontic parameters 24 such that generic factors may be used for any given patient. Further note that the generic factors may be customized for each patient and/or custom factors may be newly created for each patient. As one of average skill in the art will appreciate, as long as a digital model of the patient's orthodontic structure is available, creation of digital models of the contributing factors of the force system may be readily obtained, derived, created, etc. As one of average skill in the art will further appreciate, the force system includes the effects of bonding pad thickness, bonding agent properties, the arch wire, ligation forces, and biological characteristics (e.g., growth, tooth interference, tooth anatomy). Each of these factors may be adjusted individually or in combination to optimize the force system.

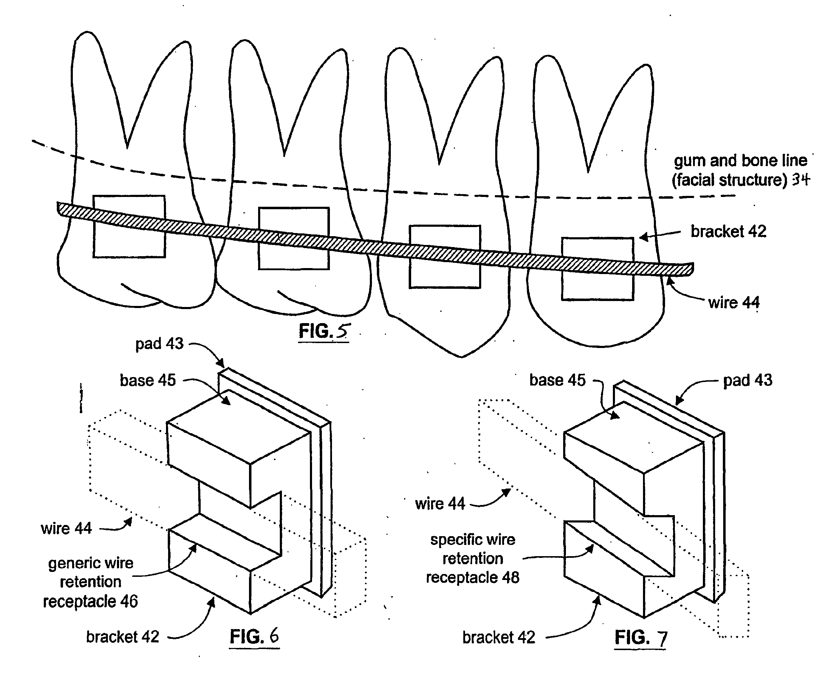

Figure 5 illustrates a graphical representation of a patient's teeth having an orthodontic apparatus attached thereto. The orthodontic apparatus includes a plurality of brackets 42 and an arch wire 44. As shown, the brackets 42 and arch wire 44 are installed below the gum and bone line 34. Throughout the treatment, the brackets are fixed to the teeth whereby the arch wire 44 is manipulated to achieve the desired orthodontic structure (i.e., the desired tooth placement) via an optimized force system. The brackets 42 may be the type of brackets illustrated in Figure 6 and 7.

Figure 6 illustrates a standard bracket or a generic prescription bracket 42 that includes a generic wire retention receptacle 46, a bonding pad 43, and a base 45. Typically the bonding pad 43 will include a wire mesh and be securely attached to the base 45. For the standard bracket, the generic wire retention receptacle 46 is a simple groove (i.e., a slot) in the bracket 42 without complex angles or depths. For the generic prescription bracket 42, often referred to as a straight arch wire bracket, the generic wire retention receptacle 46 is a groove in the bracket 42 that includes a generic angularity of complex angles and grooves that have been normalized for semi-custom treatment. The arch wire 44 is inserted into the bracket as shown to provide the desired torque or force system on the corresponding tooth.

Figure 7 illustrates a custom bracket 42 having a specific wire retention receptacle 48, a bonding pad 43, and a base 45. In this embodiment, the bracket has a retention receptacle 48 designed to include complex angles of depth and groove that are determined for a particular patient. As such, the orthodontic apparatus applied to a patient's tooth may include brackets having generic arch wire retention receptacles 46 or brackets having specific wire retention receptacles 48.

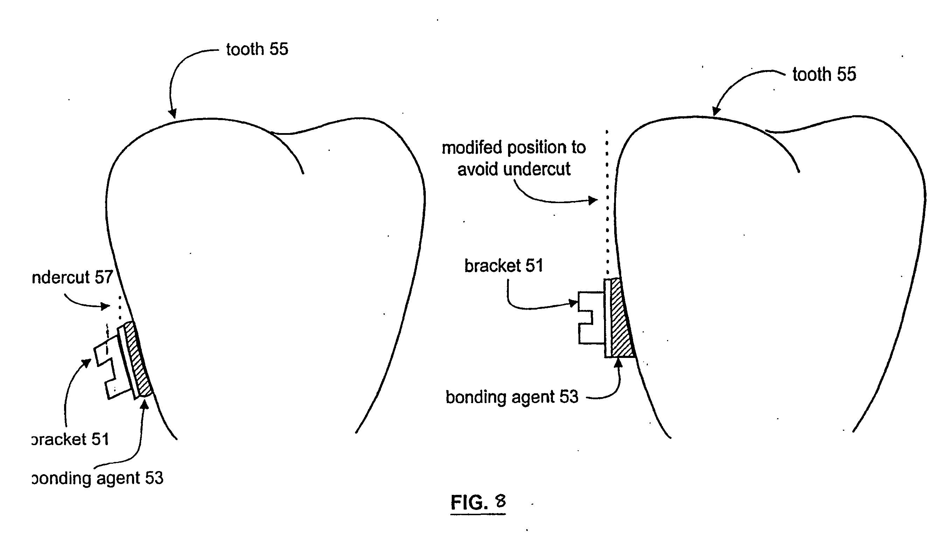

Figure 8 illustrates a graphical representation of a correction for an undercut condition. As is known, when the bracket 51 is placed on the tooth 55 such that a vertical line from the bracket intersects with the tooth 55, an undercut 57 condition exists. When an undercut 57 condition exists; it is difficult to install the bracket using a transfer tray, since, when the transfer tray is removed after the bracket as been bonded, it must be pulled off at an angle. By pulling the tray off at an angle, there is a risk of loosening, or pulling off, the bracket 51. To avoid the undercut 57 condition, the bonding agent thickness may be varied to provide the same force system as the bracket placement with the undercut 57. However, by modifying the position of the bracket, the undercut condition is avoided as are the associated difficulties.

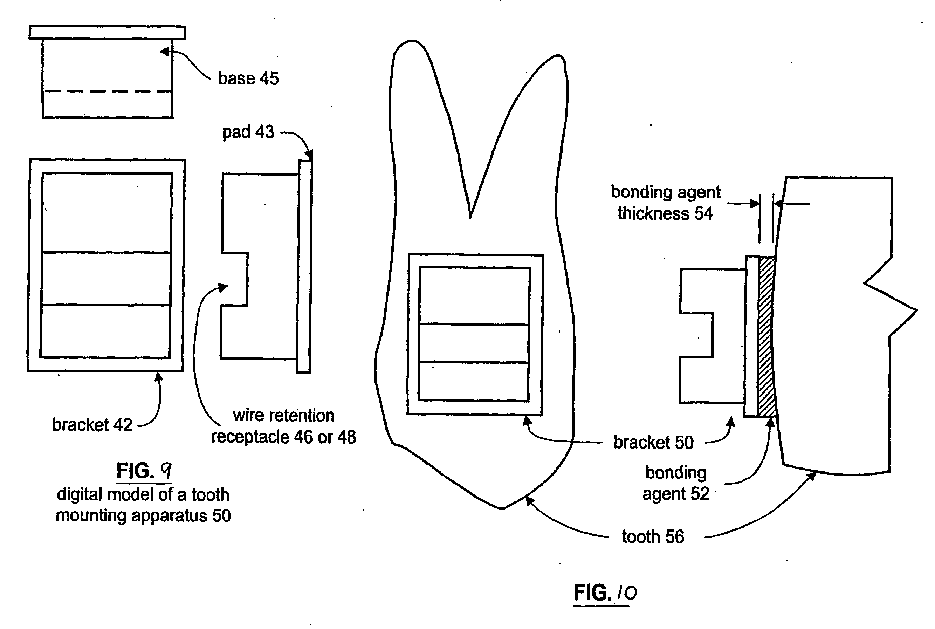

Figure 9 illustrates a three-dimensional digital model of a tooth mounting apparatus 50. The tooth mounting apparatus 50 may be a bracket, band, head gear tube, or any device used to mount onto a patient's tooth for reception of a displacement apparatus such as an arch wire and/or head gear. The three-dimensional digital model of the tooth mounting apparatus 50, hereinafter referred to generically as a bracket, may be scanned into the database 24, generated by a computer graphics designer, or transferred from a pre-existing library of the digital images. As shown, the bracket includes a bonding pad, a height, width and depth, and a slot for receiving the arch wire.

Figure 10 illustrates a graphical representation of a three-dimensional model of a given tooth having a bracket fixed thereto. The tooth 56 has a bracket 50 mounted to it via a bonding agent 52. The bonding agent 52 may be an adhesive, cement, and/or any agent used within orthodontics to adhere a bracket to a tooth. The bracket is offset from the tooth by the bonding agent thickness 54. The particular example of Figure 10 is generated digitally within the server 14 to simulate the desired tooth movement. Co-pending U. S. Patent Application No. 09/451,609 entitled "Method and Apparatus for Determining Tooth Movement in Accordance with an Ideal Orthodontic Structure" having a filing date of November 30,1999 describes the process of digitally determining the force systems on a patient's teeth using brackets, bonding agents thickness, arch wires, etc. to achieve desired results. Once the digital model of a tooth having the bracket positioned thereon in accordance with the desired positioning is derived, an orthodontic template may be generated.

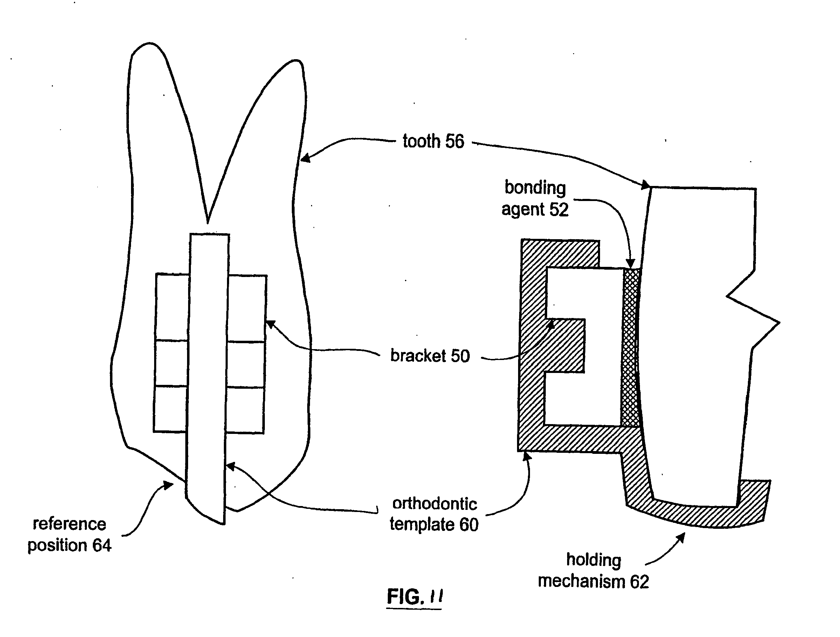

Figures 11-15 illustrate various embodiments and/or features of orthodontic templates. But, prior to discussing such embodiments and/or features, a general discussion of orthodontic templates will be presented. Such orthodontic templates may be used as a carrier system and/or as a bracket/base transfer tray that inserts into the carrier system. The carrier system is comprised of a generic carrier and individual bracket fixtures housed by the carrier and can either house bracket bases, brackets, or both. The bracket fixture references tooth digital models and locates patient tooth geometry (as discussed with reference to Figure 1), which is obtained by using 3D capture and mapping technology such as video imaging, scanning, and ultrasound to create electronic tooth data. Bracket electronic data can either be scanned or created electronically to combine bracket and tooth electronic data. Additional factors of template design include, but are not limited to, material characteristics of the template (e. g., tear strength, peel strength), undercuts, and design features (e. g., singular template or compound template).

The tooth bracket trays may contain multiple or individual brackets for placement and bonding. The tooth bracket trays fit into the generic carrier allowing flexibility to use generic brackets while still referencing relative to tooth topology. The tooth bracket can be inserted into the generic bracket carrier using either a male or female engagement or locking mechanism. The bracket carrier may contain a 3D window, which allows specific tooth bracket and bracket base trays to be inserted. Additionally, the carrier can have the brackets imbedded in the carrier without a window.

The tooth bracket tray is created to fit over individual or multiple teeth using the digital tooth model and a method of creating a physical fixture from the data. The bracket tray can locate using individual or multiple teeth on the crown in the occlusal, lingual, or labial areas on the tooth. The purpose of the carrier is a global fixture for holding the bracket/base trays. The purpose of the trays is to provide proper bracket and base location on the tooth surfaces during positioning and bond curing. By providing occlusal and labial references from multiple teeth, the bracket is able to rest in the patient's mouth without additional fixtures and engage the bracket base to the tooth surface.

The bracket trays would be created either directly from the tooth data using numerically controlled manufacturing or rapid prototyping methods such as NC machining or stereo-lithography or indirectly using a male model of the teeth and brackets created by NC controlled methods as mentioned above and vacuum-forming or another process to create a fixture from the physical tooth and bracket model. The carrier and trays can be composite or clear material to allow UV curing of adhesives through the fixture. Additionally, an NC robot can place brackets into the bracket tray using 3D tooth and bracket data.

The carrier system and trays can have a capillary or "reservoir" system so that the excess bond material can fill into this area when pressure is applied to the fixture during tooth engagement. This prevents excess bond material from building up between the bracket and the tooth, allowing more accurate placement and less variability in the overall bonding process. In addition, fiber optics may be added to the carrier such that light may be used to cure the brackets.

In addition, the orthodontic template may be generated as a bracket tray using a male tooth model with representation of the brackets in the model. The male tooth model with bracket representation is created referencing 3D tooth data and 3D bracket data using 3D capture and mapping technology such as video imaging, scanning, and ultrasound. The bracket data can be either scanned in using above-described technologies or generated directly within the 3D data from published profiles. The female tray (impression) is created using vacuum-forming or other techniques for creating a fixture from a physical male model. Alternatively, the female tray may be created directly by using 3D tooth and bracket data and utilizing NC machining or stereo-lithography techniques.

Further, the carrier system and trays can provide direct environments for optimal bonding. The carrier can provide either suction or dispersion to the teeth through a series of capillaries throughout the fixture. The capillaries act as fluid channels to provide either suction of air and liquid or dispersion of air and liquid to and from the teeth. Relevant liquid may include but not be limited to primers, adhesives, sealants, water, and saliva.

Still further, custom bracket bases may be created using 3D tooth data contoured to fit the patient's teeth. This concept involves creating bracket bases individually, which can have custom surfaces on the tooth side and the bracket side, and permanently installing them on the patient's teeth and interlocking the bracket to the base after bonding the base. Tooth geometry is provided by using 3D capture and mapping technology such as video imaging, scanning, and ultrasound. A base carrier fixture can be created to hold custom bases during base installation and can also be created referencing 3D tooth data. Custom bases can contain specific features to provide an adhesive "reservoir" inset into the base for uniformity of bond thickness and strength and increase post bonding positional accuracy. The base tray allows the bases to be installed in the patient's mouth and the brackets subsequently bonded to the base. The base tray can reference either individual or multiple teeth and can place one or more bases at a time. The base tray can also be composite or made of transparent materials to allow UV light to pass through for adhesive curing.

Yet further, bracket placement verification can be provided at any step of orthodontic treatment using the 3D capture and mapping technology. Additional techniques such as non-destructive testing can be used to verify bond strength of the bracket bond on each tooth.

In addition, an orthodontic bracket without a base may be replaced with a unique adhesive bonding surface. The bracket and tooth will have individual bond layers and the bracket base will not be present. An interlocking mechanism or other interface is attached to both the tooth and the bracket individually to allow engagement and disengagement of the bracket to the base during bracket installation. By separating the pad and the bracket, the pad is allowed more flexibility in terms compensating for adhesive thickness, materials, and surface characteristics of both sides of the bracket base. Both the pad and bracket location can be verified using the 3D capture and mapping technology. As such, only the pad could be sent to the orthodontist or a prebonded bracket may be sent.

Still further, shaping of the bracket adhesive to the tooth configuration may be done to improve bonding and location. This can be provided by removing excess adhesive and cutting to a specific adhesive footprint shape through conventional or machine cutting techniques prior to installation. An additional method for custom adhesive shaping is to "grow" or deposit material using NC, deposition, or rapid prototyping techniques referencing 3D tooth and bracket data. The adhesive preform is cut or deposited to a specific shape based on 3D tooth data. Due to current curing techniques of the adhesive on the bracket, this requires a UV resistant package for transporting the bracket.

As such, any one of the following benefits, among others, are obtained singularly or in combination:
(1) Quicker and more accurate placement of brackets due to fixture single or multiple tooth references created from accurate 3D tooth data.
(2) Improved bond strength and improved accuracy provided by custom bases and adhesive shaping based on accurate 3D tooth data. The bases, the bonding agent, and adhesive pads may be shaped to the tooth topology using the 3D tooth data.
(3) Electronic generation and data representation of both patient's teeth and orthodontic brackets.
(4) Adhesive reservoir provided on the base or bracket to provide uniform bond thickness and strength.
(5) Adhesive reservoir on bracket/base tray and carrier to allow bond material runoff to provide uniform bond thickness and improve positional location.
(6) Bracket placement validation during any step using 3D scanning. Verification of bond strength of brackets and bases using non-destructive testing technology.
(7) Closed loop real-time location feedback system during bracket positioning.
(8) Transparent carrier and bracket/base tray material for UV or laser curing of bonding agents.
(9) Bases are bonded to the patient's tooth or teeth either independently or using a carrier/base tray without placing brackets. Brackets are connected to bases with an interlock method.
(10) UV resistant transporting package may be used.
(11) Elimination of bracket base using and interlocking bracket and tooth for attachment method. The locking mechanism is individually bonded to both the tooth and the bracket.
(12) The carrier contains a 3D window that allows specific tooth bracket trays to be inserted.
(13) The bracket tray may contain multiple or individual brackets for placement and bonding and references single or multiple tooth geometry from electronic tooth data.
(14) Placement of brackets in the bracket tray using a NC robot referencing 3D tooth and bracket data or a stereo lithographic model.
(15) The carrier may also be windowless and have the bracket impression embedded in the carrier. The brackets can be inserted manually or through NC controlled robotics.
(16) The carrier and bracket tray can have a capillary system which can remove and supply gases and liquids such as sealant, primers, and water suction to provide an improved environment for bracket location and bonding.
(17) Female bracket tray using a male tooth model with actual or simulated brackets in place can be used.
(18) Male tooth model created from 3D tooth and bracket data can be used.
(19) Female bracket tray created using 3D tooth and bracket data.
(20) The orthodontist may be provided a mold of the orthodontic structure with the orthodontic apparatus installed such that he/she may create a template.
(21) Can control undercuts prior to placement of the brackets into the template.

Figure 11 illustrates a graphical representation of one particular orthodontic template 60. In this illustration, the tooth has the bracket 50 ready to be bonded thereto by the bonding agent 52. The orthodontic template 60 has a receptacle for holding the bracket in place and a corresponding holding mechanism 62 that corresponds to a reference position 64 on the tooth. As such by having the digital image of the tooth, the properties of the tooth, i.e., contour shape, unique geometries, etc. is digitally known. Utilizing this information as a reference position 64 for the orthodontic template, the holding mechanism 62 may be readily derived. By having the holding mechanism 62 of the orthodontic template 60 customized to the reference position 64 of a given tooth (i.e., the unique shape of the tooth or a portion thereof), the bracket can be accurately positioned in accordance with the digital modeling of bracket placement.

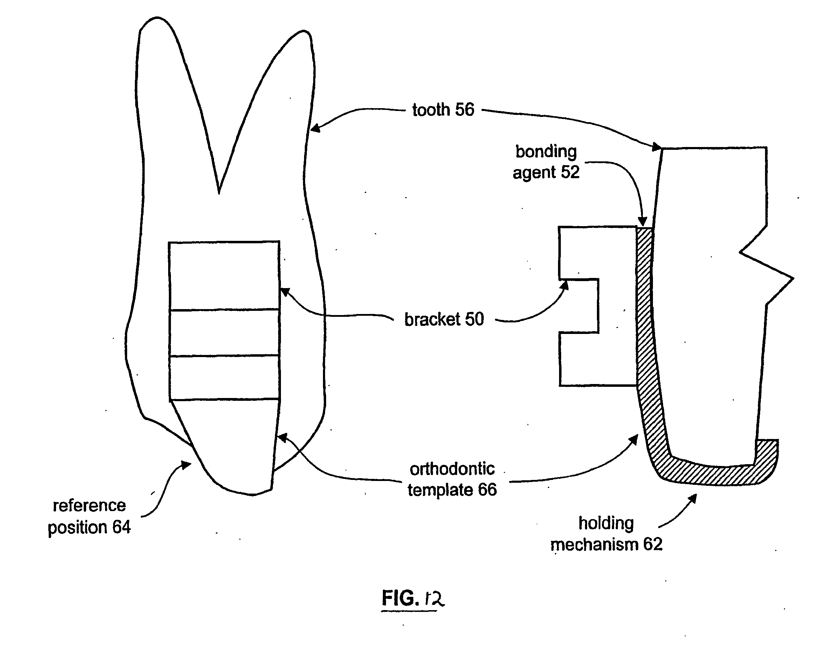

Figure 12 illustrates an alternate orthodontic template 66. In this embodiment of an orthodontic template 66, the bonding agent 52 is extended to produce the orthodontic template 66. The bonding agent includes a holding mechanism 62 that corresponds to the referenced position 64 of the given tooth 56. In this embodiment, once the tooth is positioned, the bonding agent is activated to adhere the bracket to the tooth and the excess bonding agent is removed. As such, no additional parts are required as in the example of Figure 11.

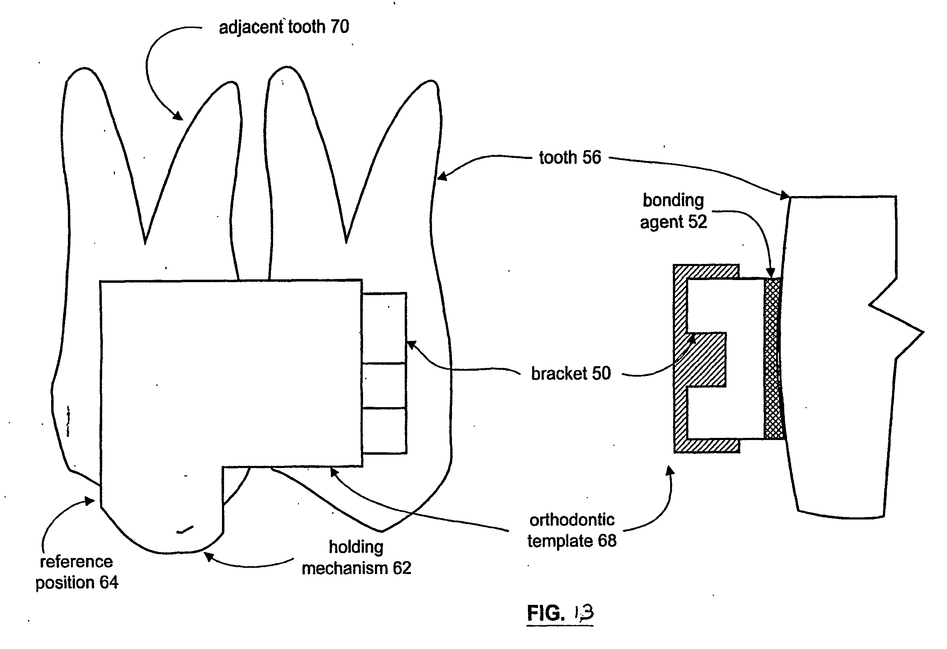

Figure 13 illustrates another embodiment of an orthodontic template 68. In this embodiment, the referenced position 64 is on an adjacent tooth 70. The holding mechanism 62 of orthodontic template 68 is then designed in accordance with the reference position 64 of the adjacent tooth. Note that the adjacent tooth may be a tooth with or without a bracket already mounted. As such, an orthodontist may utilize a plurality of dependent orthodontic templates to position brackets on a patient's teeth in a sequential manner. For example, one tooth may be selected as a primary tooth that is to be the first tooth to have a bracket mounted thereon. Accordingly, the orthodontic template for the primary tooth would have a reference position that did not include a previously placed bracket. Subsequent bracket placement may include orthodontic templates that have the reference position being the placement of the bracket on the primary tooth.

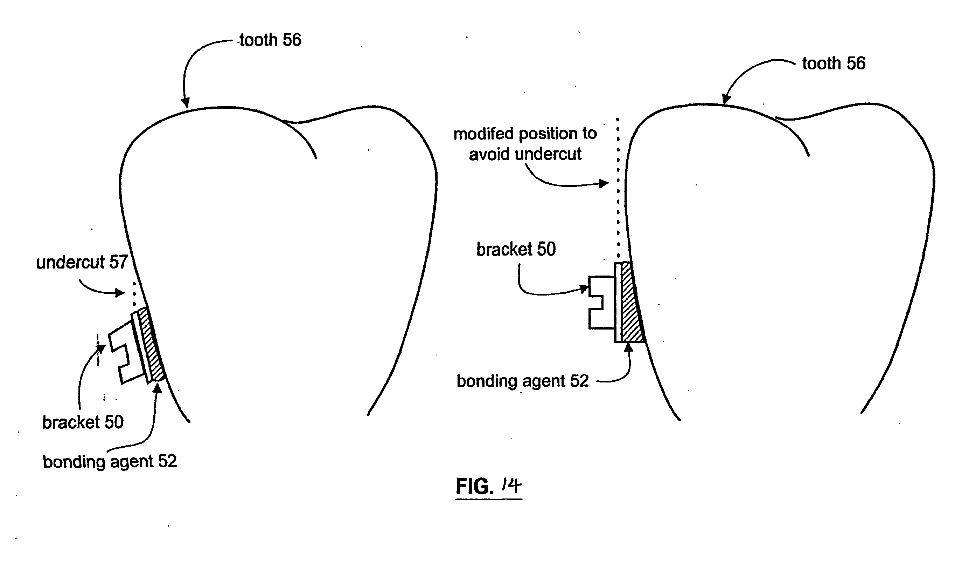

Figure 14 illustrates a graphical representation of a correction for an undercut condition. As is known, when the bracket 50 is placed on the tooth 56 such that a vertical line from the bracket intersects with the tooth 56, an undercut 57 condition exists. When an undercut 57 condition exists, it is difficult to install the bracket using a transfer tray, since, when the transfer tray is removed after the bracket has been bonded, it must be pulled off at an angle. By pulling the tray off at an angle, there is a risk of loosening, or pulling off, the bracket 50. To avoid the undercut 57 condition, the bonding agent thickness may be varied to provide the same force system as the bracket placement with the undercut 57. However, by modifying the position of the bracket, the undercut condition is avoided as are the associated difficulties.

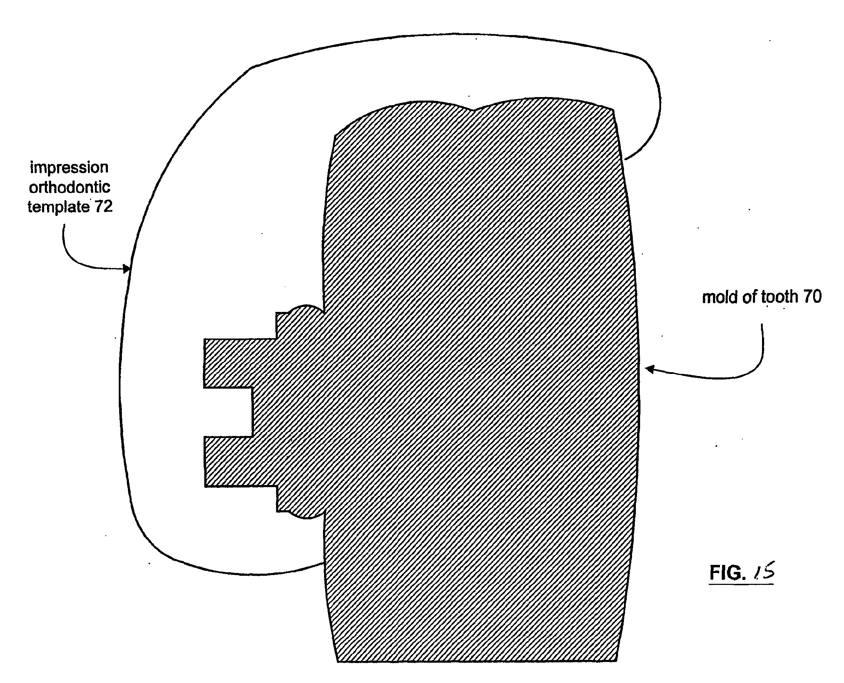

Figure 15 illustrates a graphical representation of a positive mold of a tooth 70 being fabricated from the digital image of the orthodontic structure with the orthodontic apparatus installed. Note that the orthodontic apparatus includes at least one of brackets, rapid maxillary expansion device, retainer, arch wire, bands, appliances, bonding pad, bonding agent physical properties, bonding agent adhering properties, and headgear tubes. As such, a portion of the selected orthodontic apparatus may be any one of these elements. For example, if the digital model were made with the brackets installed with a bonding agent, the positive mold would include the bracket positioning in the ideal location, along with the ideal bonding agent properties. The bonding agent properties include adherence properties (e.g., bonding strength), physical properties (e.g., height, width, depth), and type of bonding agent (e.g., UV cured, laser cured, chemical cured).

The positive mold 70 may be for a single tooth or for any number of teeth and be shipped as the orthodontic template to an orthodontist. As such, the orthodontist may fabricate the over-mold or impression orthodontic template for holding and positioning the portion of the selected orthodontic apparatus, i.e., the brackets, wires, etc. Alternatively, the over-mold may be fabricated along with the mold 70, where the combination is provided to the orthodontist as the orthodontic template.

As an alternative to fabricating the mold 70 with the orthodontic apparatus as part of the mold, a mold of the orthodontic structure, without brackets, may be made. From this mold, a robot, or electronic feedback mechanism may be used to place actual brackets on the mold. Once this is done, the over-mold may be fabricated.

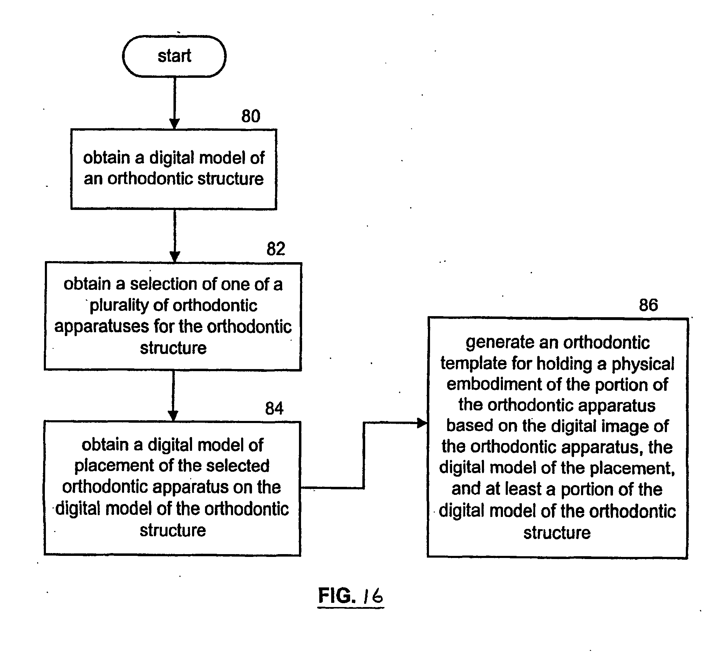

Figure 16 illustrates a logic diagram of a method for generating an orthodontic template that assists in the placement of an orthodontic apparatus. The process begins at step 80 where a digital model of an orthodontic structure is obtained. This has been previously discussed and has been referenced to a co-pending U.S. Patent Application having a serial number of 09/451,637. Note that the digital model of the orthodontic structure may be obtained by directly scanning the orthodontic structure of the orthodontic patient to obtain image data and then converting the image data into the digital model. The process then proceeds to step 82 where a selection of one of a plurality of orthodontic apparatuses is obtained for the given orthodontic structure. As previously mentioned, the database 24 may include a plurality of orthodontic appliances that the orthodontist may use to select the given apparatus to be used on a given patient. In addition, an orthodontic site system may select the digital image of the orthodontic apparatus from the database of digital representations of the orthodontic apparatuses. Having done this, the digital images would then be provided to the orthodontic server. Note that the orthodontic site system may be operated by the orthodontist and/or an employee of an orthodontist. Further note that the orthodontic apparatus may include active or passive orthodontic appliance such as brackets, rapid maxillary expansion devices, retainers, arch wires, bands, appliances; and/or head gear tubes.

The process then proceeds to step 84 where a digital model of the placement of the selected orthodontic apparatus on the digital model on the orthodontic structure is obtained. This process may be achieved utilizing the processing described in co-pending U.S. Patent Application previously mentioned having a serial number of 09/451,609. The process then proceeds to step 86 where a digital model of a tooth mounting apparatus for a given tooth is retrieved. For example, if a bracket is to be mounted on a tooth as determined in step 84, the

digital model for that bracket is obtained for the given tooth. The process then proceeds to step 88 where an orthodontic template for holding a physical embodiment of the tooth mounting apparatus is generated based on the digital image of the tooth mounting apparatus, the digital model of the placement, and at least a portion of the digital model of the orthodontic structure.

The orthodontic template may be fabricated by producing, as the orthodontic template, a physical model of the orthodontic structure with the at least a portion of the selected orthodontic apparatus placed thereon from the digital model of placement of the selected orthodontic apparatus on the digital model of the orthodontic structure. Alternatively, the orthodontic template may be generated by producing, as part of the orthodontic template, an over-mold from the physical model of the orthodontic structure with the at least a portion of the selected orthodontic apparatus placed thereon, and installing physical embodiments of the at least a portion of the selected orthodontic apparatus into the over-mold.

As another alternative, the orthodontic template may be generated by producing a physical model of the orthodontic structure from the digital model of the orthodontic structure; and automatically placing the at least a portion of the selected orthodontic apparatus on the physical model of the orthodontic structure based on the digital model of placement of the selected orthodontic apparatus on the digital model of the orthodontic structure. From this process, the generation of the orthodontic template may further include producing, as part of the orthodontic template, an over-mold from the physical model of the orthodontic structure with the at least a portion of the selected orthodontic apparatus placed thereon; and installing physical embodiments of the at least a portion of the selected orthodontic apparatus into the over-mold. As an alternative additional processing, the generation of the orthodontic template may further include producing, as part of the orthodontic template, an overmold from the physical model of the orthodontic structure with the at least a portion of the selected orthodontic apparatus placed thereon; and removing the over-mold from the physical model such that the at least a portion of the selected orthodontic apparatus is contained in the over-mold, wherein the automatic placement of the at least a portion of the selected orthodontic apparatus on the physical model of the orthodontic structure utilized a temporary bonding agent.

As yet another alternative to generating the orthodontic template, the orthodontic template may be generated by producing a mold of the placement of the at least a portion of the orthodontic apparatus on the orthodontic structure based on the digital model of the placement of the at least a portion of the orthodontic apparatus on the orthodontic structure, wherein the mold includes alignment structure corresponding to at least a portion of the orthodontic apparatus, and wherein the alignment structure aligns the at least a portion of the orthodontic apparatus for installation. The alignment structure may be location windows and/or alignment guides. Further, the alignment structure may be used to place the brackets on a mold of the patient's teeth. The mold with the brackets mounted thereon may then have an impression taken to obtain the template.

As still another alternative to generating the orthodontic template, the orthodontic template may be generated by generating a first orthodontic template for a first orthodontic appliance of the at least a portion of the selected orthodontic apparatus, wherein the first orthodontic appliance corresponds to a first tooth of the orthodontic structure; and generating a second orthodontic template for a set orthodontic appliance of the at least a portion of the selected orthodontic apparatus, wherein the set orthodontic appliance corresponds to a set of teeth of the orthodontic structure.

Once the orthodontic template is designed, it may be fabricated in accordance with the digital image for the orthodontic template. Accordingly the digital image for the orthodontic template may include programming instructions for milling, machining, 3D printing, and/or generating a mold to produce the orthodontic template. Once the orthodontic template has been produced, a physical embodiment of the bracket is installed therein and delivered to the orthodontist for installation. Once the orthodontic template is positioned on the tooth, the orthodontic template may be scanned to verify proper positioning and repositioned if necessary. If the orthodontic template is property positioned, the bracket is bonded to the tooth. The bracket positioning may also be scanned to determine that the bracket is positioned in the desired location.

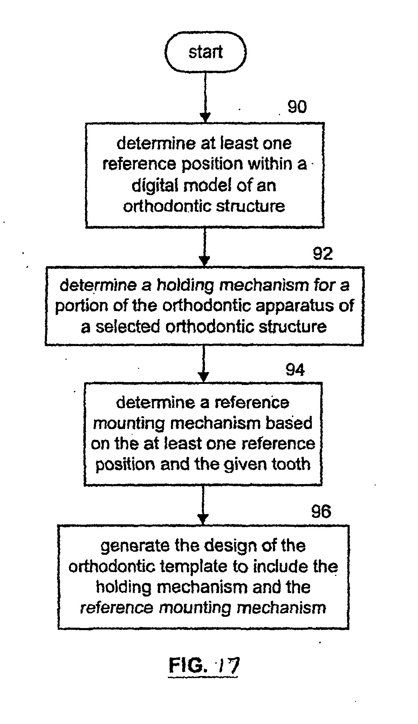

Figure 17 illustrates a logic diagram of a method for generating a orthodontic template that assists in placement of an orthodontic apparatus. The process begins at step 90 where at least one referenced position within a digital model of an orthodontic structure is determined. Note that the at least one referenced position may be a bracket on an adjacent tooth, an opposing tooth, a plurality of teeth, a plurality of brackets, and/or the given tooth itself. The process then proceeds to step 92 where a holding mechanism for a tooth mounting apparatus of a selected orthodontic structure is determined. The process then proceeds to step 94 where a referenced mounting mechanism based on the at least one reference position for the given tooth is determined. The process then proceeds to step 96 where the design of the orthodontic template is generated to include the holding mechanism and the referenced mounting mechanism. Note that if the referenced position is chosen to be the given tooth, the design of the orthodontic template includes the holding mechanism in accordance with the surface geometry of the given tooth. Alternatively, if the referenced position is an adjacent tooth, the orthodontic template is designed to include the holding mechanism in accordance with the surface geometry of the adjacent tooth. Further note that the material used to generate the orthodontic template may be the bonding agent. Alternatively, the tooth mounting apparatus may be fabricated to include the orthodontic template as an extension of the tooth mounting apparatus, wherein the orthodontic template is fabricated as a detachable placement wire.

The illustrations provided thus far have related to a single orthodontic template corresponding to a single bracket. The teachings already described are equally applicable to generating multiple orthodontic templates for multiple bracket placements sequentially and/or for generating a single orthodontic template that places multiple brackets simultaneously. To achieve this, a digital image for each corresponding bracket for a set of teeth of the orthodontic structure is retrieved. Having done this, a orthodontic template for holding a physical embodiment of each of the corresponding brackets is determined based on the digital image of the corresponding brackets, the digital model of the placement, and at least a portion of the digital image of the orthodontic structure. As mentioned, the orthodontic template may be fabricated as a single structure for parallel installations of the brackets, or generated as a orthodontic template having segregatable structures for individual installation of the corresponding brackets.

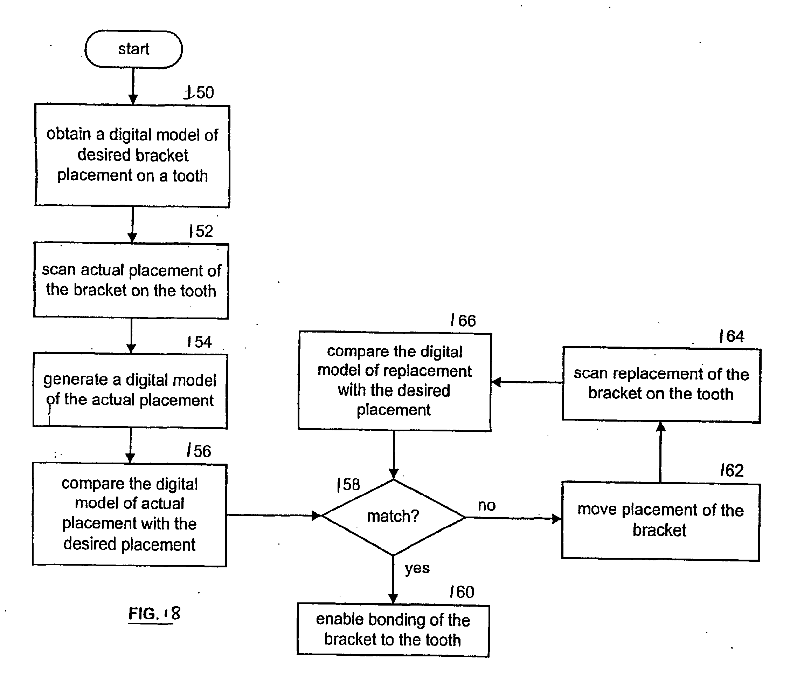

Figure 18 illustrates a logic diagram of a method for bonding a bracket to a tooth by constructing a digital orthodontic template coupled with a validation approach. The process begins at step 150 where a digital model of the desired bracket placement on a tooth is obtained. This is done utilizing the teachings of co-pending patent applications which have respective serial numbers of 09/451,609 and 09/451,564. The process then proceeds to step 152 where the actual bracket placement is scanned. Note that the initial bracket may be placed utilizing a robotic placement device in a closed-loop scanning process, or utilizing a hand-held placement device in a closed-loop scanning process. If the hand-held placement device is used, the placement process may be enhanced by displaying the scanned images on monitor 13 in a magnified manner.

The process then proceeds to step 154 where a digital model of the actual placement is generated. The process then proceeds to step 156 where the digital model of the actual placement is compared with the digital model of the desired placement. The process then proceeds to step 158 where a determination is made as to whether the actual placement substantially matches the desired placement. Substantially matching will depend on the particular type of force system desired and the accuracy in which the orthodontist wants to practice. Typically, the error (i.e., or a substantial match) will have a vector magnitude variance of less than one millimeter and an angular rotational variance of less than a few degrees. The orthodontist may determine the error based on the patient's needs. Note that the bracket may not be accurately placed for a variety of reasons including, but not limited to, the base being misaligned with respect to the bracket, the bonding agent thickness being incorrect, and/or any imperfections in the manufacture of the bracket.

If the actual placement and desired placement match, the process proceeds to step 160 where the bonding is enabled such that the bracket is permanently adhered to the tooth, where permanently refers to the duration of the patient treatment. Note that the processing steps discussed thus far may be performed on a tooth-by-tooth basis for multiple teeth. Once one tooth has been completed, the process is repeated for other teeth. Further note that the enabling of the bonding may be done by activating a light source to cure a bonding adhesive thereby securing the bracket to the tooth. The bonding adhesive may be cement, and/or any chemical adhesive used by orthodontics for such bonding purposes. As one of average skill in the art will appreciate, multiple teeth may be processed in parallel using the present teachings.

If the Actual bracket placement does not substantially match the desired bracket placement the process proceeds to step 162. At step 162 the actual placement of the bracket is moved. The movement of bracket placement may be assisted by providing corrective feedback positioning information utilizing the scanning process. For instance, the scanning process may indicate that the bracket needs to be moved down and to the left by a millimeter and rotated by an angle of 2 degrees, etc. Such a recommendation for repositioning is readily calculable from the digital model of the desired position and the digital model of the current actual placement. Having moved the bracket, the process proceeds to step 164 where the replacement positioning of the bracket is scanned. The process then proceeds to 166 where the digital image of the replacement movement of the bracket is compared with the desired placement. The process then proceeds to step 158. The process will remain in loop 158 through 166 until the actual placement substantially matches the desired placement.

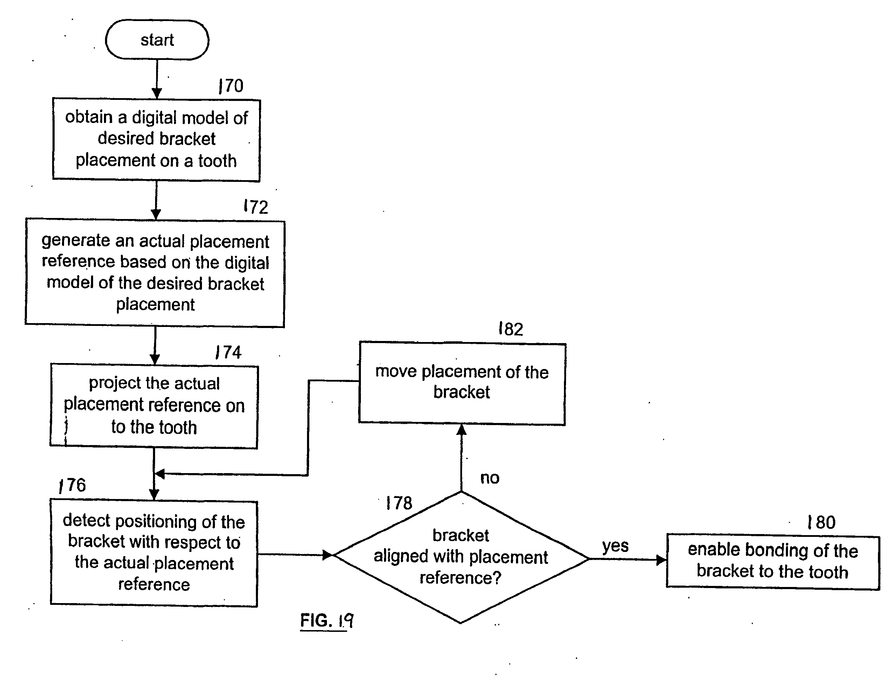

Figure 19 illustrates a logic diagram of an alternate method for bonding a bracket to a tooth. The process begins at step 170 where a digital model of a desired bracket placement on the tooth is obtained. The process then proceeds to step 172 where an actual placement reference is generated based on the digital model of the desired bracket placement. The process then proceeds to step 174 where the actual bracket placement reference is projected onto the tooth. This was illustrated with reference to Figure 3. The process then proceeds to step 176 where the positioning of the bracket is detected with respect to the actual placement reference. The process then proceeds to step 178 where a determination is made as to whether the bracket is aligned with the placement reference. If so, the process proceeds to step 180 where bonding of the bracket to the tooth is enabled.

If the bracket is not aligned with the placement reference, the process proceeds to step 182. At step 182 the placement of the bracket is moved or the arch wire is changed. The movement may be assisted by providing corrective feedback on positioning based on scanning and displaying the actual placement on a monitor or 3D simulations. The process then proceeds to step 176 and remains in loop 176, 178 and 182 until the bracket is aligned with the reference position. Note that bracket placement may be verified by simulating whether the brackets line up in three-dimensional space or whether at least some of the brackets line up. Further note that bracket placement, adhesive thickness, adjustment of arch wire, ligation forces, choice of alloy, wire cross-section are factors that may be adjusted singularly or in combination to provide the optimal force system and/or optimal placement in a closed loop system.

Using an iterative method in accordance with the present invention is advantageous over prior methods that were ultimately based upon a single two-dimensional analysis. By using a three-dimensional model in accordance with a specific embodiment of the present invention in conjunction with an iterative process, any factor that affects tooth movement (i.e. brackets, wires, adhesion, physiological changes) can be simulated to determine appropriate treatment changes. Such compensation in treatment is not possible using prior methods which were based upon assumptions from a single model that the tooth movement would progress in a known manner. Therefore, the prior art methods would specify a single static treatment based upon this assumption. If any unwanted tooth movement occurred during treatment, the specified treatment would no longer be valid, requiring changes to be made based upon a practitioner's expertise. The present system provides a dynamic system that through the use of periodic feedback, i.e. periodic three-dimensional scanning, can be monitored and adjusted as needed by the system in an efficient manner. As such, unexpected tooth movement, such as occurs when a patient does not cooperate, or through biological changes, can be readily controlled.

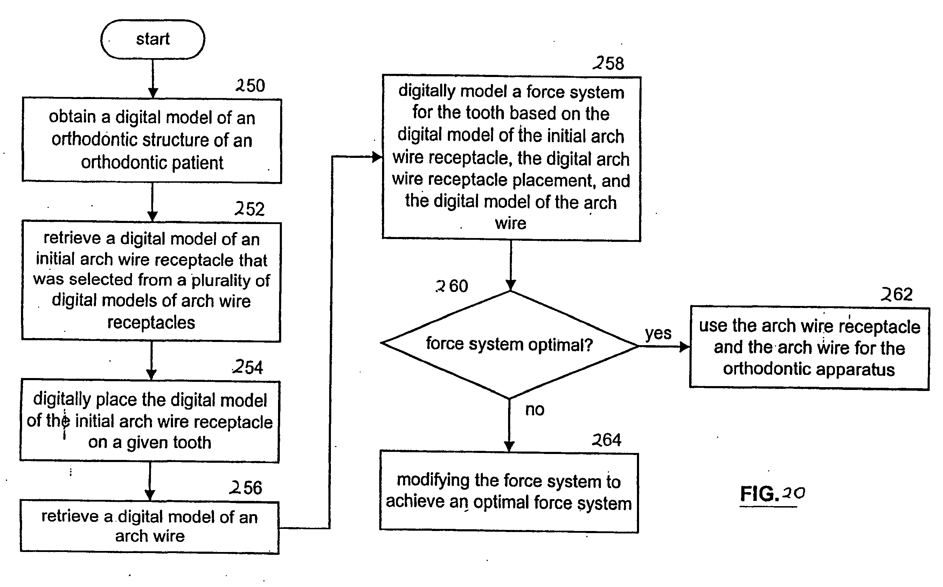

Figure 20 illustrates a logic diagram of a method for arch wire receptacle optimization. The process begins at step 250 where a digital model of an orthodontic structure of an orthodontic patient is obtained. The process then proceeds to step 252 where a digital model of an initial arch wire receptacle that was selected from a plurality of digital models of arch wire receptacles is retrieved. Note that the arch wire receptacle includes brackets, bands, and/or headgear tubes. The process then proceeds to step 254 where the digital model of the initial arch wire receptacle is digitally placed on a given tooth. The process then proceeds to step 256 where a digital model of an arch wire is retrieved. The process then proceeds to step 258 where a force system for the tooth is digitally modeled based on the digital model of the initial arch wire receptacle, the digital arch wire receptacle placement, and the digital model of the arch wire. Note that the determination of the force system may be based on a tooth to bonding agent force, a bonding agent to bracket force and a wire-to-bracket force. In essence, the force for each of these types of interactions is based on wire tensile strength, shape of the brackets, thickness of the bonding agent, patient physical parameters and is readily determined using conventional statics mathematics, which can be digitally modeled as discussed.

The process proceeds to step 260 where a determination is made as to whether the force system is optimal. If so, the process proceeds to step 262 where the arch wire receptacle and the arch wire for the orthodontic apparatus are used. If the force system is not optimal, the process proceeds to step 264 where the force system is modified to obtain an optimal force system. Modifying of the force system may include changing the bonding pad thickness, the bonding agent properties, the arch wire, ligation forces, the brackets, selecting the characteristics of the arch wire, and/or reducing the force system requirements such that given orthodontic appliances may adhere to the desired force system. The modifying of the force system may also be done by adjusting the functional loading on other teeth, adjusting the material of the initial arch wire receptacle and/or the arch wire. Alternatively, or in addition, the force system may be modified by selecting different digital models of the arch wire receptacle and/or arch wire, adjusting the placement of the digital model of the initial arch wire receptacle, adjusting the bonding agent properties, thickness, type, etc. of the digital model of the initial arch wire receptacle, and/or adjusting the bracket wire coupling of the digital model of the initial arch wire receptacle (e.g., changing the slot on the bracket or placement of the bracket).

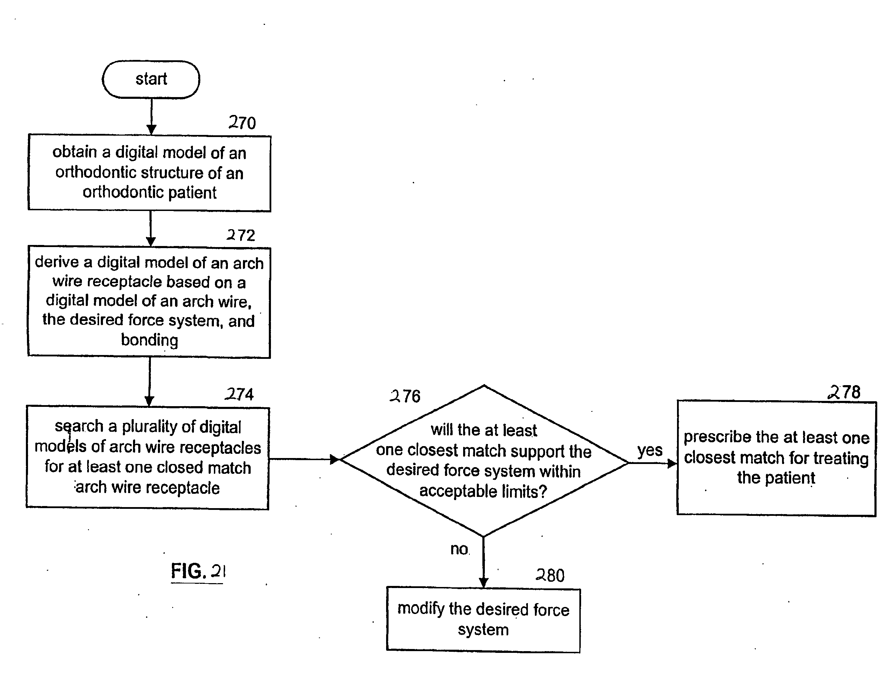

Figure 21 illustrates a logic diagram of an alternate method for arch wire receptacle optimization. The process begins at step 270 where a digital model of an orthodontic structure of an orthodontic patient is obtained. The process then proceeds to step 272 where a digital model of an arch wire receptacle is derived based on a digital model of an arch wire, the desired force system, and the bonding agent. The process then proceeds to step 274 where a plurality of digital models of arch wire receptacles is searched for a closest match arch wire receptacle. In other words, a search is performed to find an existing arch wire receptacle that achieves the desired force system. The process then proceeds to step 276 where a determination is made as to whether the closest match arch wire receptacle will support the desired force system within acceptable limits. The acceptable limits will be primarily left up to the orthodontist and such factors will be based on patient pain tolerance, treatment plan, length of treatment, costs, etc. If the closest match arch wire receptacle will support the desired force system, the process proceeds to step 278 where the closest match arch wire receptacle is prescribed for treating the patient.

If the closest match arch wire receptacle will not support the desired force system, the process proceeds to step 280. At step 280 the desired force system is modified. The desired force system may be modified by adjusting placement of the digital model of the arch wire receptacle, adjusting the bonding agent properties of the bonding of the bracket to the teeth, adjusting the bracket wire coupling (i.e., the slot of the digital model of the arch wire receptacle, bracket placement, and/or adjusting the material of the arch wire receptacle and/or of the arch wire).

The preceding discussion has presented a method for fabricating a orthodontic template. By utilizing digital imagery and digital models, a orthodontic template may be fabricated without the bracket being the focal point of orthodontic treatment. By removing the focal point from the bracket, more flexible orthodontic treatment may be obtained. The orthodontic template produced in accordance with the teachings of the present application assist in such orthodontic treatment. As one of average skill in the art will appreciate, other embodiments may be derived from the teachings of the present patent without deviating from the scope of the claims.

## Claims

1. A method for generating an orthodontic template that assists in placement of orthodontic apparatus, the method comprising the steps of:
a) obtaining a digital model of an orthodontic structure of an orthodontic patient;
b) obtaining a selection of one of a plurality of orthodontic apparatuses for the orthodontic structure;
c) obtaining a digital model of placement of the selected orthodontic apparatus on the digital model of the orthodontic structure;
d) manufacturing a physical model of the digital model obtained in step c) of the placement of the selected orthodontic apparatus on the digital model of the orthodontic structure; and
e) generating the orthodontic template by forming an overmold of said physical model and placing a physical embodiment of at least a portion of the selected orthodontic apparatus into the overmold.

2. The method of claim 1, wherein step (a) further comprises obtaining the digital model of the orthodontic structure by directly scanning the orthodontic structure of the orthodontic patient.

3. The method of claim 1, wherein step (b) further comprises:
selecting the selected orthodontic apparatus from a database of digital representations of the plurality of orthodontic apparatuses.

4. The method of claim 3, wherein the selected orthodontic apparatus further comprises at least one bracket.

5. The method of claim 1, wherein step (e) further comprises:
determining at least one reference position within the digital model of the orthodontic apparatus;
determining a holding mechanism for the at least a portion of the selected orthodontic apparatus;
determining a reference mounting mechanism based on the at least one reference position and the given tooth; and
generating a design of the orthodontic template to include the holding mechanism and the reference mounting mechanism.

6. The method of claim 5, further comprising the steps of:
determining the at least one reference position to be on the given tooth; and
generating the design of the orthodontic template to include the holding mechanism in accordance with a surface of the given tooth.

## Patentansprüche

1. Ein Verfahren zur Erzeugung einer kieferorthopädischen Vorlage, die bei der Platzierung einer kieferorthopädischen Vorrichtung hilft, wobei das Verfahren die Schritte umfasst:
a) Erhalten eines digitalen Modells eines kieferorthopädischen Aufbaus eines kieferorthopädischen Patienten;
b) Erhalten einer Auswahl von einer von mehreren kieferorthopädischen Vorrichtungen für den kieferorthopädischen Aufbau;
c) Erhalten eines digitalen Modells der Platzierung der ausgewählten kieferorthopädischen Vorrichtung auf dem digitalen Modell des kieferorthopädischen Aufbaus;
d) Herstellen eines physischen Modells des in Schritt c) erhaltenen digitalen Modells der Platzierung der ausgewählten kieferorthopädischen Vorrichtung auf dem digitalen Modell des kieferorthopädischen Aufbaus; und
e) Erzeugen der kieferorthopädischen Vorlage durch Bilden einer Umspritzung des physischen Modells und Platzieren einer physischen Ausgestaltung von mindestens einem Teil der ausgewählten kieferorthopädischen Vorrichtung in der Umspritzung.

2. Das Verfahren gemäß Anspruch 1, wobei Schritt (a) ferner Erhalten des digitalen Modells des kieferorthopädischen Aufbaus durch direktes Scannen des kieferorthopädischen Aufbaus des kieferorthopädischen Patienten umfasst.

3. Das Verfahren gemäß Anspruch 1, wobei Schritt (b) ferner umfasst:
Auswählen des ausgewählten kieferorthopädischen Modells aus einer Datenbank von digitalen Darstellungen der mehreren kieferorthopädischen Vorrichtungen.

4. Das Verfahren gemäß Anspruch 3, wobei die ausgewählte kieferorthopädische Vorrichtung ferner mindestens ein Bracket umfasst.

5. Das Verfahren gemäß Anspruch 1, wobei Schritt (e) ferner umfasst:
Bestimmen mindestens einer Bezugsposition in dem digitalen Modell der kieferorthopädischen Vorrichtung;
Bestimmen eines Haltemechanismus für den mindestens einen Teil der ausgewählten kieferorthopädischen Vorrichtung;
Bestimmen eines Bezugsbefestigungsmechanismus auf Basis der mindestens einen Bezugsposition und des bestimmten Zahns; und
Erzeugen einer Ausgestaltung der kieferorthopädischen Vorlage, um den Haltemechanismus und den Bezugsbefestigungsmechanismus einzuschließen.

6. Das Verfahren gemäß Anspruch 5, ferner umfassend die Schritte:
Bestimmen der mindestens einen Bezugsposition, die sich auf dem bestimmten Zahn befinden soll; und
Erzeugen der Ausgestaltung der kieferorthopädischen Vorlage, um den Haltemechanismus in Übereinstimmung mit der Oberfläche des bestimmten Zahns einzuschließen.

## Revendications

1. Procédé de génération d'un gabarit orthodontique qui aide à la mise en place d'un appareil orthodontique, le procédé comprenant les étapes consistant à :
a) obtenir un modèle numérique d'une structure orthodontique d'un patient orthodontique ;
b) obtenir une sélection d'un d'une pluralité d'appareils orthodontiques pour la structure orthodontique ;
c) obtenir un modèle numérique de mise en place de l'appareil orthodontique choisi sur le modèle numérique de la structure orthodontique ;
d) fabriquer un modèle physique du modèle numérique obtenu à l'étape c) de la mise en place de l'appareil orthodontique choisi sur le modèle numérique de la structure orthodontique ; et
e) générer le gabarit orthodontique en formant un surmoule dudit modèle physique et placer une forme de réalisation physique d'au moins une portion de l'appareil orthodontique choisi dans le surmoule.

2. Procédé selon la revendication 1, dans lequel l'étape (a) comprend en outre l'obtention du modèle numérique de la structure orthodontique en balayant directement la structure orthodontique du patient orthodontique.

3. Procédé selon la revendication 1, dans lequel l'étape (b) comprend en outre :
la sélection de l'appareil orthodontique choisi dans une base de données de représentations numériques de la pluralité d'appareils orthodontiques.

4. Procédé selon la revendication 3, dans lequel l'appareil orthodontique choisi comprend en outre au moins un support.

5. Procédé selon la revendication 1, dans lequel l'étape (e) comprend en outre :
la détermination d'au moins une position de référence dans le modèle numérique de l'appareil orthodontique ;
la détermination d'un mécanisme de retenue pour la au moins une portion de l'appareil orthodontique choisi ;
la détermination d'un mécanisme de montage de référence sur la base de la au moins une position de référence et la dent donnée ; et
la génération d'un concept du gabarit orthodontique pour inclure le mécanisme de retenue et le mécanisme de montage de référence.

6. Procédé selon la revendication 5, comprenant en outre les étapes consistant à :
déterminer la au moins une position de référence qui doit se trouver sur la dent donnée ; et
générer le concept du gabarit orthodontique pour inclure le mécanisme de retenue conformément à une surface de la dent donnée.
